# FASCICULE DE BREVET EUROPEEN

(11) **EP 1 257 667 B1**
(45) Date de publication et mention de la délivrance du brevet: **19.07.2006**
(21) Numéro de dépôt: 01907845.0
(22) Date de dépôt: 20.02.2001
(51) Int. Cl.: C12Q 1/68, C12Q 1/00, G01N 27/26, C12Q 1/28

(54) **PROCEDE ELECTROCHIMIQUE DE DETECTION D'ACIDES NUCLEIQUES**
ELECTROCHEMISCHES VERFAHREN ZUM NACHWEIS VON NUKLEINSÄUREN
ELECTROCHEMICAL METHOD FOR DETECTING NUCLEIC ACIDS

(30) Priorité: 24.02.2000 FR 0002323
(43) Date de publication de la demande: 20.11.2002
(73) Titulaire: ARGENE, 09120 Varilhes (FR)
(72) Inventeur: AZEK, Fatima, F-94410 Saint-Maurice (FR); BROSSIER, Pierre, F-21800 Quetigny (FR); JOANNES, Martine, F-78420 Carrieres-sur-Seine (FR); LIMOGES, Benoît, F-91220 Bretigny-sur-Orge (FR)
(74) Mandataire: Texier, Christian
(86) Numéro de dépôt international: PCT/FR2001/000488
(87) Numéro de publication internationale: WO 2001/062953

(56) Documents cités:
- EP-A- 0 663 446
- EP-A- 0 671 625
- WO-A-85/02627
- WO-A-86/03837
- WO-A-86/05815
- WO-A-93/20230
- WO-A-95/24641
- WO-A-97/13870
- WO-A-99/07879
- GB-A- 2 276 724
- AZEK F ET AL: "Hybridization assay at a disposable electrochemical biosensor for attomole detection of amplified human cytomegalovirus DNA" ANALYTICAL BIOCHEMISTRY, vol. 284, 15 août 2000 (2000-08-15), pages 107-13, XP002176118

## Description

La présente invention concerne un nouveau procédé de détection et/ou de dosage de molécules d'acides nucléiques dans un échantillon biologique par électrochimie, ainsi qu'une trousse de réactifs permettant de mettre en oeuvre ce procédé. Un mode particulier de mise en oeuvre de ce procédé permet de détecter la présence de contamination par un agent pathogène dans un échantillon biologique.

La présence d'agents pathogènes dans un organisme peut aujourd'hui être détectée par plusieurs méthodes. La plupart mettent en oeuvre une étape préalable d'amplification, en général par réaction de polymérisation en chaîne (PCR), qui permet d'amplifier de manière spécifique des fragments d'ADN du génome viral. Cette méthode extrêmement sensible permet de détecter un très faible nombre de molécules dans l'organisme et, sous certaines conditions, de quantifier le nombre de copies du génome de l'agent présentes initialement.

Cette technique est *a priori* facile d'utilisation et permet d'obtenir des résultats fiables.

Les méthodes traditionnellement utilisées pour analyser les produits de PCR sont l'électrophorèse, avec coloration de l'ADN avec du bromure d'éthidium (BET), ou des tests d'hybridation, en utilisant des sondes marquées par exemple avec des composés radioactifs, luminescents ou encore détectables en colorimétrie. Ces techniques d'hybridation sont largement utilisées pour les diagnostics médicaux.

On a développé récemment d'autres méthodes pour l'obtention de produits de PCR qui soient directement susceptibles d'analyse. Par exemple, on peut utiliser des amorces marquées de façon spécifique, le signal émis par les fragments amplifiés étant par la suite analysé en utilisant des systèmes relativement pesants. En particulier, les amorces servant pour l'amplification peuvent porter un fluorophore dont la mesure de l'émission de fluorescence permettra de déterminer la quantité d'ADN amplifié. Par ailleurs, une limitation de ces méthodes reste la difficulté de les mettre en oeuvre facilement, dans la mesure où elles nécessitent l'utilisation d'équipements lourds. Par ailleurs, le risque d'interférences limite encore ces méthodes.

L'ADN possédant des bases nucléiques électroactives, on a également développé des systèmes de détection électrochimiques tirant partie de cette propriété pour détecter directement l'ADN hybridé, sans avoir à faire intervenir un marqueur. De façon générale, l'ADN est immobilisé sur une électrode, et la différence de courant électrique mesuré avant et après hybridation est liée à la quantité d'ADN fixé sur l'électrode. La mise en oeuvre d'un tel procédé est décrite dans la demande de brevet WO 93/20230. Cependant, cette détection directe sans marqueur n'est pas très sensible.

Afin d'améliorer encore la sensibilité, d'autres approches ont été développées faisant intervenir une molécule sonde électroactive ou un marqueur électroactif. Ainsi. PaJanti *et al*. (1996, Analytical Letters, 29, pp2309-31) décrivent différents composés électroactifs, pouvant s'associer avec l'ADN et ainsi être détectés par oxydation ou réduction en appliquant un potentiel à l'électrode. Des complexes de métaux de transition, des antibiotiques, des colorants d'acridine ou de benzamide et d'autres intercalants de l'ADN ont ainsi été employés. Ces sondes ou marqueurs électroactifs possédant de meilleures propriétés d'oxydoréduction que l'ADN, leur utilisation permet d'obtenir un rapport signal / bruit supérieur et une meilleure sensibilité. Le seuil de détection de l'ADN d'un virus de l'immunodéficience humaine de type 1, obtenu avec de telles méthodes, était de l'ordre de la nanomole (Wang *et al*., 1996. *Analytical Chemistry*, 68, 2629-34).

WO86/03837 décrit un procédé de détection et/ou de dosage d'acides nucléiques dans un échantillon, comprenant les étapes de fixation d'un acide nucléique sur des électrodes, hybridation spécifique d'un acide nucléique complémentaire à l'acide nucléique fixé couplé à une enzyme, et ajout d'un substrat de ladite enzyme de telle façon que l'action de ladite enzyme sur ledit substrat mène à la formation d'un composé électroactif qui peut être détecté par la mesure de la variation de courant faradique après application d'un potentiel à l'électrode.

La présente invention permet d'améliorer la sensibilité d'une détection électrochimique de l'ADN grâce à l'utilisation d'un marqueur enzymatique capable de transformer rapidement à la surface de l'électrode un substrat inactif en un composé électrochimiquement détectable.

Ainsi, la présente invention a pour objet un procédé de détection et/ou de dosage d'acides nucléiques dans un échantillon, comprenant les étapes suivantes effectuées dans de petits volumes compris entre 5 et 50 µl :
a. la fixation d'un acide nucléique par adsorption spécifique sur des électrodes sérigraphiées avec une encre à base de carbone,
b. l'hybridation spécifique d'un acide nucléique complémentaire à l'acide nucléique fixé, ledit acide nucléique complémentaire contenant un agent de reconnaissance,
c. l'ajout d'un agent complémentaire à l'agent de reconnaissance de (b), ledit agent complémentaire étant couplé à une enzyme,
d. l'ajout d'un substrat de ladite enzyme de telle façon que l'action de ladite enzyme sur ledit substrat mène à la formation d'un composé électroactif qui peut être détecté par la mesure de la variation de courant faradique après application d'un potentiel à l'électrode.

Lors de l'étape d, on peut avoir une cascade de réactions enzymatiques avant formation du composé électroactif. Si des enzymes différentes sont couplées à l'agent complémentaire défini à l'étape c., le composé obtenu après action de la première enzyme sur le substrat ajouté peut lui-même être substrat d'une autre enzyme, et ainsi de suite, jusqu'à obtenir enfin le composé électroactif.

La mesure de courant peut être effectuée à partir de techniques électrochimiques telles que la voltammétrie linéaire, cyclique, impulsionnelle, à impulsion différentielle, à vague carrée, ou encore l'ampérométrie, la chronoampérométrie, la coulométrie, la chronocoulométrie, la potentiométrie par redissolution anodique ou cathodique.

L'acide nucléique fixé sur l'électrode peut être l'acide que l'on cherche à détecter (cible) ou peut être une sonde. Dans ce cas, l'acide nucléique cible est ajouté ultérieurement. Il est marqué par l'agent de reconnaissance. On peut effectuer un tel marquage, par exemple lors d'une amplification en particulier par PCR, en utilisant des amorces marquées.

Comme précisé ci-dessus, l'acide nucléique cible peut avoir été amplifié, en particulier par PCR. L'acide nucléique que l'on adsorbe à la surface de l'électrode est de préférence sous forme simple brin, soit qu'il le soit naturellement, soit qu'il soit un acide nucléique double brin dénaturé, afin de permettre l'hybridation de l'acide nucléique complémentaire. Un tel acide nucléique double brin dénaturé est également considéré comme étant simple brin au sens de l'invention. Si l'acide nucléique cible est double brin, l'hybridation est entendue comme la formation d'un complexe d'acide nucléique triple hélice.

Une « sonde » se définit, au sens de l'invention, comme étant un fragment d'acide nucléique simple brin ou un fragment double brin dénaturé comprenant par exemple de 12 à quelques kilobases, notamment de 15 à quelques centaines de bases, de préférence de 15 à 50 ou 100 bases, possédant une spécificité d'hybridation dans des conditions déterminées pour former un complexe d'hybridation avec un acide nucléique cible.

Par « acide nucléique », on entend en particulier de l'ADN, de l'ARN, ou des PNA. Cet acide nucléique peut être sous forme simple brin ou sous forme double brin. Il peut également être modifié, en particulier au niveau des liaisons entre les différents éléments. En particulier, on peut penser à des liaisons phosphorothioates, plutôt que phosphodiesters. Il peut également être marqué, radioactivement, ou par des composés fluorescents, luminescents ou par des organométalliques.

Par « agent de reconnaissance », on entend un composé susceptible d'être couplé aux acides nucléiques et d'être reconnu spécifiquement par un autre composé, que l'on appelle agent complémentaire. Des exemples d'agents de reconnaissance et complémentaire qui peuvent être utilisés comprennent en particulier les complexes antigène - anticorps, haptène - anticorps ou biotine - streptavidine ou avidine. Ces derniers agents seront préférés pour la mise en oeuvre du procédé selon l'invention.

Par « échantillon biologique », on entend tout échantillon contenant du matériel biologique. Ceci comprend en particulier les cultures cellulaires maintenues *in vitro*, ou les échantillons qui peuvent être obtenus à partir d'un animal ou d'un humain (biopsies, échantillons sanguins).

La Demanderesse a démontré que le procédé selon l'invention permet d'obtenir un seuil de sensibilité de détection d'acides nucléiques, en particulier de l'ADN amplifié, extrêmement faible, de l'ordre de l'attomole. En effet, cette méthode a l'avantage par rapport aux méthodes précédemment décrites de posséder plusieurs étapes d'amplification :
- l'étape d'amplification de l'ADN par PCR lorsque celle-ci est effectuée.
- une étape dite d'amplification enzymatique, lors de l'ajout du substrat de la peroxydase. En effet, la variation de courant faradique mesurée sera due à la concentration de composé électroactif présente dans la solution, ladite concentration pouvant être modifiée en jouant sur le temps d'incubation substrat / enzyme,
- l'éventuelle étape de cascade enzymatique telle que décrite plus haut.

Par ailleurs, une molécule d'agent complémentaire peut être couplée à plusieurs molécules d'enzymes, ce qui est une source supplémentaire d'amplification du signal.

La détection de l'acide nucléique par le procédé selon l'invention s'effectue en effet par la détection d'un composé électrochimique plutôt que par la détection de l'acide nucléique, et est plutôt basée sur l'amplification d'un signal plutôt que sur l'amplification de la cible.

Par ailleurs, le procédé selon l'invention peut aisément être miniaturisé et/ou automatisé, ce qui réduit les risques de contamination des échantillons, et permet d'effectuer des analyses à un coût réduit. Il est même avantageux d'effectuer une telle miniaturisation.

La Demanderesse a en effet montré que, de façon surprenante, la sensibilité du système est améliorée lorsque l'on travaille dans de petits volumes. Par petits volumes, on entend des volumes compris entre quelques microlitres et quelques dizaines de inicrolitres, en particulier de 5 à 50 µl, de manière préférée de 10 µl.

En effet, dans la mesure où l'on détecte la variation de courant faradique à la surface de l'électrode, il est avantageux d'augmenter le rapport S/V (Surface de l'électrode / Volume de la solution), afin d'obtenir un meilleur signal.

Les électrodes seront des électrodes sérigraphiées avec une encre à base de carbone, modifiées ou non. De telles électrodes ont été précédemment décrites dans l'état de la technique, par exemple dans la demande de brevet WO 93/20230 ou dans Bagel *et al*. (1997, Analytical Chemistry, 69, pp4688-94). En particulier, on utilise des électrodes sérigraphiées, avec une encre qui contient du carbone et des dérivés styrèniques. Un dérivé préféré est le polystyrène. Le ratio (en poids) graphite/polystyrène est compris entre 1/10 et 10/1, de préférence entre 1/5 et 5/1, de façon encore plus préférée entre 1/2 et 2/1. On préfère tout particulièrement un rapport entre 5/4 et 7/4, en particulier 3/2. Le solvant utilisé doit permettre une bonne homogénéisation des composés présents dans l'encre et pouvoir « sécher » rapidement (environ 30 minutes à 3 heures), en particulier par évaporation. On préfère que l'évaporation s'effectue à température ambiante. On effectue de préférence la sérigraphie sur des feuilles flexibles de polyester ou de PVC. Ces descriptions correspondent à des exemples particuliers d'électrodes, mais il est entendu que l'homme du métier saura optimiser celles-ci en fonction de l'usage recherché.

L'invention est en particulier caractérisée par le fait que les acides nucléiques présents dans la solution que l'on analyse (qui peut être un échantillon biologique) sont adsorbés de manière spécifique sur l'électrode.

Pour ce faire, on utilise un tampon de fixation, caractérisé en ce qu'il contient 1,5M d'acétate d'aminonium. Ledit tampon peut en particulier être à base de PBS (4,3mM NaH₂PO₄ ; 15,1mM Na₂HPO₄ ; 50mM NaCl, pH 7,4) ou de Tris (50mM Tris ; 1mM MgCl₂.6H₂O 50mM NaCl, pH 7,4).

Lors de l'ajout de la sonde marquée avec l'agent de reconnaissance, il est préférable que celle-ci ne se fixe qu'à l'ADN cible, et qu'elle ne se fixe pas de manière non spécifique aux électrodes. En effet, une telle fixation mènerait à la liaison ultérieure de l'agent complémentaire et à la formation du composé électroactif, après ajout du substrat de l'enzyme. On obtiendrait alors une réaction faussement positive.

Le tampon d'hybridation doit donc permettre l'hybridation spécifique de la sonde marquée à l'ADN cible. On utilise des tampons d'hybridation classiques, tels que décrits dans Sambrook et al. (Molecular cloning : a laboratory manual. 1989. 2^{nd} Edition. Cold Spring Harbor Laboratory Press, Cold Spring Harbor, NY, USA, voir en particulier p9.54). Un tampon d'hybridation utilisable dans le procédé selon l'invention contient du 6 X SSC, 0.1% SDS.

De préférence, si on utilise une enzyme seule, celle-ci possède une activité d'oxydase. Par exemple, elle peut être une peroxydase, une glucose oxydase, mais ce peut être aussi un autre type d'enzyme telle une hydrolase comme la phosphatase alcaline. De façon préférée, on utilise une peroxydase, en particulier la peroxydase de raifort (Horseraddish peroxydase, HRP). Un substrat préféré de la peroxydase liée à la streptavidine sera l'ortho-phénylènédiamine (OPD). La peroxydase catalyse l'interaction de l'OPD avec H₂O₂ pour donner un composé coloré électroactif soluble dans l'eau : le 2.2'-diaminoazobenzène (DAA). On peut toutefois utiliser d'autres substrats de la peroxydase, par exemple la tétraméthylbenzydine (TMB), les dérivés de l'o-phénylènediamine et diaminobenzènes, l'hydroquinone et ses dérivés, l'acide 2,2'-azinobis(3-éthylbenzothiazoline-6-sulfonique), les phénoxazines et analogues, les systèmes 4-aminoantipyrine / phénol ou 4-aminoantipyrine / aniline, les ferrocènes et analogues.

Lorsque l'on effectue la détection après une cascade enzymatique, il est important que la dernière enzyme en jeu transforme le dernier substrat en composé électroactif. Les enzymes précédentes, qui servent à l'amplification du signal peuvent être des enzymes classiques utilisées en biologie, et actives dans les conditions expérimentales. Des enzymes avantageuses sont par exemple les enzymes qui permettent l'hydrolyse des sucres, comme les glucosidases et apparentées.

L'invention concerne également une trousse de réactifs, pour la mise en oeuvre du procédé selon l'invention qui contient :
a. un tampon de fixation contenant 1,5 M d'acétate d'ammonium permettant la fixation de l'acide nucléique sur les électrodes sérigraphiées avec une encre à base de carbone
b. un tampon d'hybridation permettant l'hybridation spécifique de l'acide nucléique complémentaire sur l'acide nucléique fixé sur les électrodes,
c. un agent de reconnaissance afin de marquer l'acide nucléique complémentaire,
d. un agent complémentaire à l'agent de reconnaissance (c), couplé à une enzyme, et
e. un substrat de ladite enzyme, menant à la formation d'un composé électroactif permettant une mesure de la variation de courant faradique de la solution.

On peut rajouter d'autres éléments, en particulier des amorces pour l'amplification de l'acide nucléique cible, ou un tampon permettant la dénaturation de l'acide nucléique double brin dans le cas où l'acide nucléique de départ est double brin et l'on fixe un acide nucléique simple brin sur les électrodes.

Le procédé selon l'invention peut être mis en oeuvre pour la détection et/ou le dosage d'ADN provenant de sources variées. En particulier, on peut citer la détection d'ADN d'origine bactérienne, virale ou cellulaire.

On peut en effet utiliser le procédé selon l'invention pour détecter la surexpression ou la sous-expression de certains gènes impliqués dans des phénomènes cancéreux, par exemple après des étapes de transcription inverse - amplification à partir d'ARN messager qui peut être préparé à partir d'une biopsie. En effet, le procédé selon l'invention permet la quantification de l'acide nucléique cible, à condition de disposer d'un étalon interne.

On peut également mettre en oeuvre le procédé selon l'invention pour détecter et/ou quantifier d'éventuelles contaminations bactériennes, que ça soit dans des échantillons alimentaires (en particulier les contaminations à *Salmonella, Listeria, E. coli e*ntérohémorragiques O157 et/ou O11...). Le procédé selon l'invention est également d'une grande utilité pour la détection et le diagnostic d'infections bactériennes chez l'homme ou en médecine vétérinaire. On peut citer les infections à *M. tuberculosis* qui pourront être détectées à partir de sputum humain, ou la caractérisation d'autres infections pour lesquelles on veut un résultat rapide et fiable.

Le procédé selon l'invention est particulièrement utile pour la détection de virus dans l'organisme, permettant d'obtenir une excellent sensibilité, et donc de détecter un nombre très réduit de copies d'ADN viral. En particulier, on peut détecter la présence d'un virus dans un échantillon biologique en suivant un protocole comprenant les étapes suivantes :
a. une amplification spécifique de l'ADN de l'échantillon biologique, de préférence par PCR en utilisant des amorces spécifiques du virus recherché.
b. l'isolement et l'analyse de l'ADN obtenu après amplification par un procédé selon l'invention ou en utilisant une trousse selon l'invention.

Le procédé selon l'invention permet également de détecter en même temps la présence dans un échantillon d'acides nucléiques provenant de sources ou organismes variés. En effet, le principe du procédé selon l'invention est la détection d'un courant faradique spécifique du composé électrochimique formé par l'action de l'enzyme sur le substrat.

Lorsque l'on veut détecter la présence d'acide nucléique provenant de différentes sources dans un échantillon, on peut suivre le procédé suivant :
a. on effectue l'amplification optionnelle et la fixation des acides nucléiques cibles. desdits organismes différents sur les électrodes sérigraphiée avec une encre à base de carbone
b. on effectue l'hybridation avec les acides nucléiques complémentaires aux cibles, chacun d'entre eux étant lié à un agent de reconnaissance différent,
c. on ajoute les agents complémentaires aux agents de reconnaissance, couplés à des marqueurs différents,
d. on ajoute les différents substrats des enzymes afin de générer les différents composés électroactifs,
e. on mesure le courant faradique correspondant aux différents composés électroactifs, à la surface de l'électrode, en appliquant le potentiel spécifique de chaque composé.

Les marqueurs différents de l'étape c sont des marqueurs enzymatiques ou autres. Les substrats des enzymes génèrent différents composés électroactifs, les autres marqueurs étant des marqueurs redox spécifiques. Les acides nucléiques cibles fixés sur les électrodes sont de préférence simple brin.

La génération du courant faradique étant liée à la présence du composé électroactif spécifique, on peut donc en déduire la présence ou l'absence des acides nucléiques dans l'échantillon de départ.

Il est entendu que l'exemple de procédé donné ci-dessus peut être modifié, par exemple en fixant les sondes spécifiques des différents acides nucléiques sur les électrodes, et en couplant les acides nucléiques cibles avec les différents agents de reconnaissance, par exemple lors d'une étape d'amplification par PCR.

Ce procédé permet donc d'identifier rapidement et aisément les contaminants microbiens ou viraux dans des échantillons alimentaires, ou dans un échantillon biologique.

Pour la mise en oeuvre du procédé selon l'invention lors de l'analyse d'un échantillon biologique, il est en général conseillé d'effectuer une amplification spécifique préalable de l'ADN que l'on cherche à détecter. On peut effectuer la réaction PCR directement sur l'échantillon, ou après purification préalable de l'ADN de l'échantillon. On choisit l'une ou l'autre technique en fonction de la quantité d'échantillon dont on dispose et des buts recherchés par la personne mettant en oeuvre le procédé selon l'invention. L'homme du métier connaît les techniques à employer pour isoler de l'ADN d'un échantillon biologique.

Dans le cas où le procédé est mis en oeuvre dans un système automatisé et/ou miniaturisé, on peut effectuer l'isolement de l'ADN directement sur les électrodes, par exemple en utilisant l'enseignement du brevet WO 97/41219, l'ADN ainsi isolé pouvant alors être ultérieurement amplifié par PCR.

### DESCRIPTION DES FIGURES

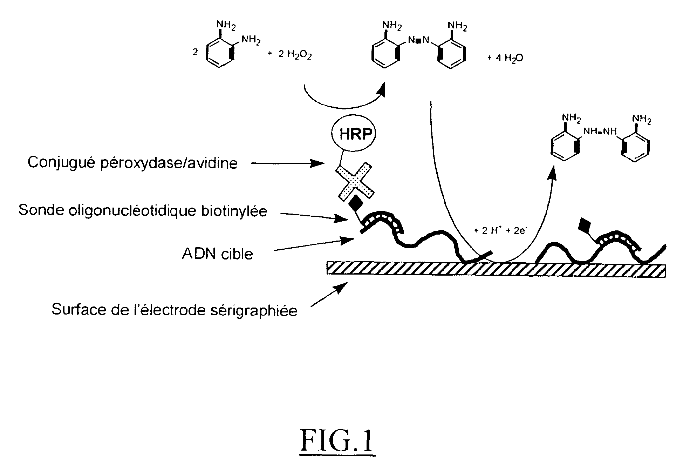

Figure 1 : Représentation schématique du procédé de détection selon l'invention sur électrode sérigraphiée.
Figure 2 : Voltammogrammes obtenus avec le procédé selon l'invention sur des électrodes revêtues de (a) l'ADN amplifié du HCMV (4.10⁹ copies dans la solution) et (b) de l'ADN amplifié du gène ETS2.
Figure 3 : Courbes de calibration (S/B, Signal / Bruit) de l'ADN amplifié du HCMV par plusieurs méthodes. On utilise une échelle logarithmique.
   - a-c : hybridation sur électrode, avec détection colorimétrique (a), ou électrochimique (b, c).
   - d : hybridation et détection conventionnelle colorimétrique sur plaque de microtitration
   - e : quantification de l'ADN par fluorescence du BET à partir d'une électrophorèse sur gel d'agarose.
Figure 4 : Etude comparative de la spécificité du procédé selon l'invention, par détection électrochimique (noir), spectrophotométrique (blanc) sur électrodes, ou par la méthode colorimétrique conventionnelle (gris). On utilise des fragments amplifiés du gène ETS2, de virus EBV et HCV, et un contrôle positif et un contrôle négatif de l'ADN amplifié du HCMV. Echelle logarithmique.
Figure 5 : Etude comparative de la capacité de détection de l'ADN amplifié du HCMV dans des échantillons humains (1-10), par la méthode conventionnelle colorimétrique sur microplaques (blanc) ou le procédé selon l'invention (noir). On a inclut un contrôle positif (+) et deux contrôles négatifs (-). Echelle logarithmique.

### EXEMPLES

### Exemple 1 : Extraction de l'ADN

L'ADN du HCMV est extrait à partir de la lignée cellulaire embryonnaire humaine de fibroblastes de poumon MRC5 infectée par la souche virale AD169, avec une trousse commerciale d'extraction d'ADN, selon les recommandations du fabricant. Cette technique est connue de l'homme du métier, et divers fabricants proposent des trousses permettant une telle extraction. On a observé, en particulier, que les trousses Invisorb, de Invitek ou QiaAmpBlood de Qiagen, permettent d'obtenir de bons résultats.

Les cellules sont lysées, adsorbées sur de la silice puis lavées par centrifugation. L'ADN est élué dans un tampon approprié et le support est éliminé. L'ADN peut ensuite être amplifié.

### Exemple 2: Amplification de l'ADN de HCMV par PCR

On utilise les amorces AC1 (SEQ ID NO 1) et AC2 (SEQ ID NO 2) qui amplifient un fragment de 406 paires de bases d'une région conservée située dans la région HIND III X du génome US du cytomégalovirus (Drouet *et al*., 1993, *J Virol Methods*, 45, 259-76).

On effectue les réactions de PCR selon les techniques habituelles connues de l'homme du métier, sur une matrice d'ADN telle que préparée dans l'exemple 1. On effectue 35 cycles possédant les caractéristiques suivantes : dénaturation à 92°C - 15 sec, hybridation à 55°C - 30 sec, extension à 72 °C - 30 sec. L'étape de dénaturation est de 7 min pour le premier cycle, et on suit l'étape d'extension du dernier cycle par un maintien de la température de 72°C pendant 2 minutes additionnelles.

On inclue un contrôle négatif pour chaque série d'expériences, qui ne contient pas de matrice d'ADN.

### Exemple 3: Quantification de l'ADN et préparation d'une gamme de concentration d'ADN de HCMV amplifié

On fait des dilutions en séries de l'ADN amplifié selon l'exemple 2, et on les étudie sur gel d'agarose avec du BET, en présence d'une quantité d'ADN calibrée. On trouve donc que la concentration de l'ADN amplifié est de 10,5 pmol/ml, ce qui correspond à 6,3.10¹² copies/ml.

On fabrique une gamme (de 6.3 10⁴ à 6,3 10¹² copies/ml) par des dilutions en série de la solution concentrée d'ADN amplifié, dans le contrôle négatif de la PCR.

### Exemple 4: Hybridation sur électrodes et détection électrochimique ou colorimétrique

La détection de l'ADN par le procédé selon l'invention se fait en quatre étapes :
a. immobilisation de l'ADN cible
b. hybridation de la sonde marquée
c. incubation du conjugué enzymatique
d. apport du substrat de détection
e. détection voltammétrique ou colorimétrique du produit généré par l'enzyme.

On dénature 2 µl d'ADN amplifié dans un milieu alcalin (soude 0,4M) à température ambiante, pendant 10 min.

On ajoute ensuite 300 µl du tampon de fixation contenant 1,5M d'acétate d'ammonium et on immerge les électrodes. On laisse incuber à 37°C pendant une nuit.

On lave ensuite les électrodes avec de l'eau distillée, et on les fait incuber pendant 30 min à 37°C dans un tampon d'hybridation (6 X SSC, 0.1% SDS) contenant 100 ng/ml de sonde AC3 spécifique de la séquence amplifiée du HCMV (SEQ ID NO 3), biotinylée.

Puis on effectue un cycle de lavage, qui consiste en 5 incubations de 1 minute dans 500 µl de solution de lavage préparée fraîchement (6 X SSC, 1% SDS).

On fait ensuite incuber les électrodes pendant 15 min à température ambiante, dans 100 µl de tampon (100mM Tris HCl pH 7,5 - 50mM NaCl - 5g/l lait écrémé) qui contient le conjugué streptavidine-peroxidase (1,6 Unités/ml), puis on effectue immédiatement un cycle de lavage, tel que décrit précédemment.

On immerge alors l'électrode dans 50 µl d'une solution de substrat OPD (40mM acide citrique. 150mM Na₂HPO₄, 5mM NaCl, 0,02% H₂O₂, une pastille d'OPD (Argène-Biosoft) dans 10 ml de tampon), et on incube à température ambiante, dans le noir pendant 30 min.

Le produit de réaction hydrosoluble, coloré et électroactif, 2,2'-diaminoazobenzène est détecté par spectrophotométrie d'absorption et par voltammétrie différentielle à impulsion (DPV), afin de comparer les deux méthodes.

Pour la lecture spectrophotométrique, on retire les électrodes et on effectue la lecture des puits à 492 nm.

Pour la lecture par DPV, on utilise une électrode de Pt comme contre-électrode et une électrode Ag/AgCl comme pseudo-électrode de référence. On utilise un potentiostat µ-Autolab (de EcoChemie) relié à une interface sur PC, en utilisant le logiciel GPSE 3 (EcoChemie). On effectue la DPV avec une hauteur d'impulsion de 25 mV, une marche de potentiel de mV, une durée d'impulsion de 0,05 sec, et un intervalle entre deux impulsions de 0,5 sec.

Pour chaque série d'expériences, on inclue 2 contrôles négatifs (tous les réactifs, mais pas d'ADN). Ainsi, on divise les valeurs optiques ou de courant par les valeurs obtenues pour ce contrôle, et on considère que les échantillons sont positifs lorsque le rapport réponse / blanc est supérieur à 2.

On obtient les résultats suivants :

### a. Utilisation du procédé selon l'invention pour la détection d'ADN

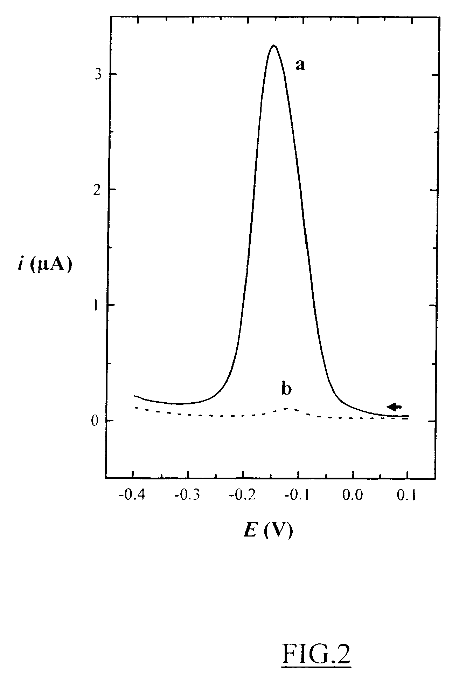

La figure 2 montre les pics enregistrés par DPV obtenus pour une électrode mise en contact avec l'ADN du HCMV amplifié (figure 2a) ou pour le contrôle négatif (figure 2b). Cette figure montre sans ambiguïté que le procédé selon l'invention permet de détecter de l'ADN présent en solution, par DPV.

### b. Spécificité, reproductibilité du procédé selon l'invention

Afin de mesurer la spécificité et la reproductibilité du procédé selon l'invention, on a comparé les pics obtenus avec 30 électrodes recouvertes, soit par de l'ADN amplifié du HCMV, soit par de l'ADN amplifié d'un gène humain ETS2. La sonde AC3 étant spécifique du HCMV, elle ne doit pas se lier à l'ADN du gène ETS2. Il n'y aura donc pas de réaction enzymatique, et en conséquence, on ne devrait pas observer de pic de courant pour le gène ETS2.

On obtient les valeurs de courant suivantes :

| | | |
|---|---|---|
| HCMV : | 3300 ± 100 nA | (écart type 3%) |
| ETS2 : | 61 ± 12 nA | (écart type 19%) |

Ceci démontre que le procédé est reproductible et que, dans le tampon spécifique d'hybridation utilisé, la sonde ne se lie pas de façon passive aux électrodes, mais se lie aux séquences complémentaires déjà adsorbées sur les électrodes.

### c. Limites de détection du procédé selon l'invention. Comparaison avec la méthode colorimétrique

On fait varier la concentration d'ADN amplifié du HCMV dans la gamme de 0,1 à 10⁶ attomoles (6,3.10⁴ à 6.3.10¹¹ copies/ml). On utilise les propriétés du produit généré par la réaction enzymatique pour comparer les méthodes voltamétrique et colorimétrique.

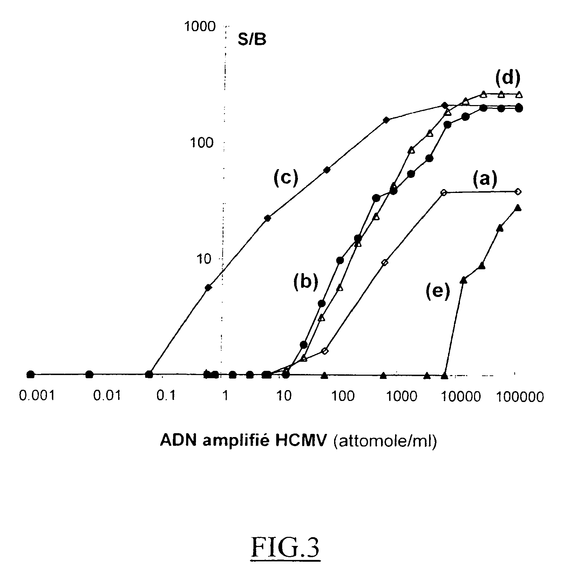

Les figures 3a et 3b montrent les courbes obtenues avec les méthodes colorimétrique et électrochimique, respectivement.

On effectue également une comparaison avec d'autres méthodes : colorimétrie d'après la trousse commerciale Hybridowell^{™} (ArgèneBiosoft) (3d), ou densitométrie de fluorescence sur gel d'agarose (3e).

On observe que la courbe de calibration (3b) de l'ADN amplifié de HCMV est linéaire, dans le domaine 50-2000 attomoles (3.10⁷ molécules d'ADN amplifié), ce qui permet une détection d'environ 10 fois moins de molécules d'ADN que par la méthode colorimétrique dans les mêmes conditions (3a). La sensibilité de la méthode est de toutes façons bien supérieure à la fluorescence sur gel d'agarose (figure 3e, limite de détection de 14 femtomoles), et est équivalente aux systèmes colorimétriques sur plaques de microtitration (figure 3d).

Afin d'augmenter la sensibilité du procédé selon l'invention, on a réduit le volume de substrat OPD apporté pour la détection (10 µl au lieu de 50 µl). La courbe obtenue est indiquée sur la figure 3c, et on observe que la limite de détection est alors portée à 0,6 attomoles (3,6.10⁵ molécules d'ADN amplifié). Ceci est 83 fois plus sensible que la technique colorimétrique sur plaques de microtitration.

Cependant, dans les expériences qui suivent, on conserve le volume de 50µl pour la solution de substrat.

### d. Sélectivité, spécificité du procédé selon l'invention

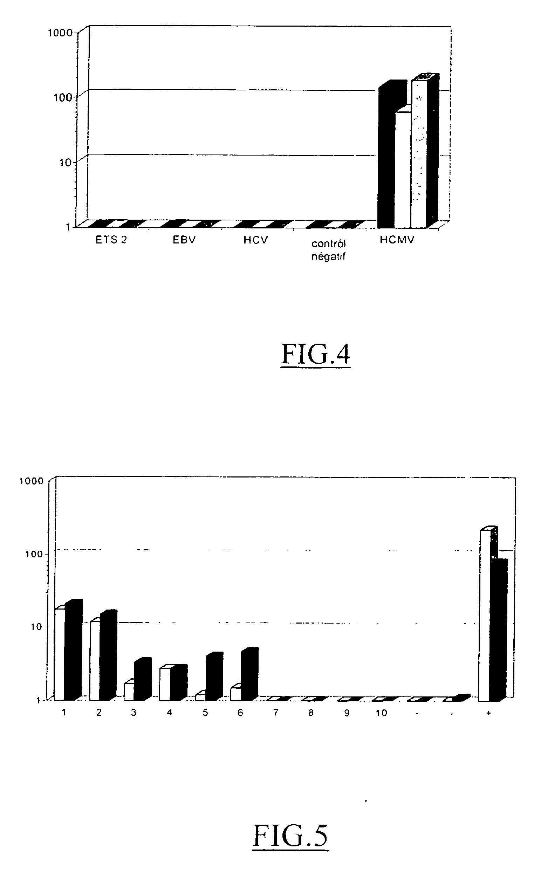

On a utilisé différents ADN, pour juger de la spécificité du procédé selon l'invention, les résultats étant présenté sur la figure 4. On a effectué une comparaison avec les deux autres méthodes colorimétriques, celle décrite dans la présente invention, et la méthode sur plaques de microtitration. Les ADN utilisés sont l'ADN amplifié du HCMV, et l'ADN du gène humain ETS2, et les ADN viraux du virus d'Epstein-Barr (EBV) ou de l'hépatite C (HCV).

On observe que la méthode permet une détection spécifique et sélective de l'ADN du HCMV, lorsque la sonde spécifique de cet ADN est utilisée, ce qui confirme que cette sonde ne s'adsorbe pas de façon passive sur les électrodes lors de l'étape d'hybridation.

### e. Caractérisation d'échantillons cliniques

On applique le procédé selon l'invention à 10 échantillons de sérum humain (4 négatifs, 6 positifs, tels que déterminé précédemment par PCR quantitative et qui possèdent de 2 à 99 copies/µl d'ADN du HCMV avant amplification), sur lesquels on a procédé à l'amplification telle que décrite dans l'exemple 2.

On compare les résultats obtenus avec le procédé selon l'invention à ceux obtenus par la méthode colorimétrique conventionnelle dans des plaques de microtitration.

La figure 5 montre que le procédé selon l'invention ne donne pas de faux positifs ou de faux négatifs, les résultats obtenus pour tous les échantillons examinés étant en conformité avec ce qui était attendu.

On observe par ailleurs que le procédé selon l'invention est au moins aussi sensible que la méthode colorimétrique conventionnelle, et que, dans le cas où le nombre de copies initiales est bas, le procédé selon l'invention permet d'obtenir un meilleur rapport signal / bruit (échantillons 5 et 6).

## Revendications

1. Procédé de détection et/ou de dosage d'acides nucléiques dans un échantillon, comprenant les étapes suivantes effectuées dans de petits volumes compris entre 5 et 50 µl :
a. la fixation d'un acide nucléique par adsorption spécifique sur des électrodes sérigraphiées avec une encre à base de carbone,
b. l'hybridation spécifique d'un acide nucléique complémentaire à l'acide nucléique fixé, ledit acide nucléique complémentaire contenant un agent de reconnaissance,
c. l'ajout d'un agent complémentaire à l'agent de reconnaissance de (b), ledit agent complémentaire étant couplé à une enzyme,
d. l'ajout d'un substrat de ladite enzyme de telle façon que l'action de ladite enzyme sur ledit substrat mène à la formation d'un composé électroactif qui peut être détecté par la mesure de la variation de courant faradique après application d'un potentiel à l'électrode.

2. Procédé selon la revendication 1, **caractérisé en ce qu'**il comporte une cascade de réactions enzymatiques lors de l'étape d), avant formation du composé électroactif.

3. Procédé selon l'une quelconque des revendications 1 ou 2, **caractérisé en ce que** l'acide nucléique simple brin fixé sur les électrodes est l'acide nucléique cible.

4. Procédé selon la revendication 3, **caractérisé en ce qu'**il comporte en outre une étape d'amplification avant l'étape de fixation de l'acide nucléique sur les électrodes.

5. Procédé selon l'une quelconque des revendications 1 à 4, **caractérisé en ce que** l'étape a) de fixation est effectuée avec un tampon de fixation contenant 1,5 M d'acétate d'ammonium.

6. Procédé selon la revendication 5, **caractérisé en ce que** l'encre pour la sérigraphie contient un mélange de carbone et de dérivés styréniques.

7. Procédé selon l'une quelconque des revendications 1 à 6, **caractérisé en ce que** l'enzyme permettant la formation du composé électroactif est une oxydase.

8. Procédé selon la revendication 7, **caractérisé en ce que** l'oxydase est une peroxydase.

9. Procédé selon la revendication 8, **caractérisé en ce que** la peroxydase est la peroxydase de raifort.

10. Procédé selon l'une quelconque des revendications 8 ou 9, **caractérisé en ce que** le substrat de la peroxydase est l'ortho-phénylènediamine OPD.

11. Trousse de réactifs pour la mise en oeuvre d'une procédé selon l'une quelconque des revendications 1 à 10, **caractérisée en ce qu'**elle contient :
a. un tampon de fixation contenant 1,5 M d'acétate d'ammonium permettant la fixation spécifique de l'acide nucléique sur des électrodes sérigraphiées avec une encre à base de carbone,
b. un tampon d'hybridation permettant l'hybridation spécifique de l'acide nucléique complémentaire sur l'acide nucléique fixé sur des électrodes,
c. un agent de reconnaissance afin de marquer l'acide nucléique complémentaire,
d. un agent complémentaire à l'agent de reconnaissance (c), couplé à une enzyme, et
e. un substrat de ladite enzyme, menant à la formation d'un composé électroactif permettant une mesure de la variation de courant faradique de la solution.

12. Procédé pour la détection de virus dans un échantillon biologique, **caractérisé en ce qu'**il comprend les étapes suivantes :
a. une amplification spécifique de l'ADN de l'échantillon biologique, de préférence par PCR en utilisant des amorces spécifiques du virus recherché,
b. l'isolement et l'analyse de l'ADN obtenu après amplification par un procédé selon l'une quelconque des revendications 1 à 10, ou en utilisant une trousse selon la revendication 11.

13. Procédé de détection de plusieurs organismes différents dans un échantillon **caractérisé en que** qu'il comprend les étapes suivantes effectuées dans de petits volumes compris entre 5 et 50 µl :
a. on effectue l'amplification optionnelle et la fixation des acides nucléiques cibles desdits organismes différents par adsorption spécifique sur l'électrode sérigraphiée avec une encre à base de carbone,
b. on effectue l'hybridation avec les acides nucléiques complémentaires aux cibles, chacun d'entre eux étant lié à un agent de reconnaissance différent,
c. on ajoute les agents complémentaires aux agents de reconnaissance couplés à des marqueurs enzymatiques différents,
d. on ajoute les différents substrats des enzymes afin de générer les différents composés électroactifs,
e. on mesure le courant faradique correspondant aux différents composés électroactifs, à la surface de l'électrode, en appliquant le potentiel spécifique de chaque composé.

## Claims

1. Method for detecting and/or assaying nucleic acids in a sample, comprising the following steps carried out in small volumes of between 5 and 50 µl:
a. attaching a nucleic acid by specific adsorption onto electrodes screen-printed with a carbon-based ink,
b. specifically hybridizing a nucleic acid complementary to the attached nucleic acid, said complementary nucleic acid containing a recognition agent,
c. adding an agent complementary to the recognition agent of (b), said complementary agent being coupled to an enzyme,
d. adding a substrate for said enzyme such that the action of said enzyme on said substrate leads to the formation of an electroactive compound which can be detected by measuring the variation in faradic current after application of a potential to the electrode.

2. Method according to Claim 1, **characterized in that** it comprises a cascade of enzymatic reactions in step d., before formation of the electroactive compound.

3. Method according to either one of Claims 1 and 2, **characterized in that** the single-stranded nucleic acid attached to the electrodes is the target nucleic acid.

4. Method according to Claim 3, **characterized in that** it also comprises an amplification step before the step of attachment of the nucleic acid to the electrodes.

5. Method according to any one of Claims 1 to 4, **characterized in that** the attaching step a) is carried out with an attaching buffer containing 1.5 M ammonium acetate.

6. Method according to Claim 5, **characterized in that** the ink for the screen printing contains a mixture of carbon and styrene derivatives.

7. Method according to any one of Claims 1 to 6, **characterized in that** the enzyme which allows the formation of the electroactive compound is an oxidase.

8. Method according to Claim 7, **characterized in that** the oxidase is a peroxidase.

9. Method according to Claim 8, **characterized in that** the peroxidase is horseradish peroxidase.

10. Method according to either one of Claims 8 and 9, **characterized in that** the substrate for the peroxidase is ortho-phenylenediamine, OPD.

11. Kit of reagents, for carrying out a method according to any one of Claims 1 to 10, **characterized in that** it contains:
a. an attaching buffer containing 1.5 M ammonium acetate for specifically attaching the nucleic acid to electrodes screen-printed with a carbon-based ink,
b. a hybridization buffer for the specific hybridization of the complementary nucleic acid to the nucleic acid attached to the electrodes,
c. a recognition agent in order to label the complementary nucleic acid,
d. an agent complementary to the recognition agent (c), coupled to an enzyme, and
e. a substrate for said enzyme, leading to the formation of an electroactive compound which allows the variation in faradic current of the solution to be measured.

12. Method for detecting viruses in a biological sample, **characterized in that** it comprises the following steps:
a. specific amplification of the DNA of the biological sample, preferably by PCR using primers specific for the virus being sought,
b. isolation and analysis of the DNA obtained after amplification using a method according to any one of Claims 1 to 10 or using a kit according to Claim 11.

13. Method for detecting several different organisms in a sample, **characterized in that** it comprises the following steps carried out in small volumes of between 5 and 50 µl:
a. optional amplification and attachment of the target nucleic acids of said different organisms by specific adsorption onto the electrode screen-printed with a carbon-based ink,
b, hybridization with the nucleic acids complementary to the targets, each one of them being linked to a different recognition agent,
c. addition of the agents complementary to the recognition agents, coupled to different enzymatic labels,
d. addition of the various substrates for the enzymes in order to generate the various electroactive compounds,
e. measurement of the faradic current corresponding to the various electroactive compounds, at the surface of the electrode, by applying the potential specific for each compound.

## Patentansprüche

1. Verfahren zum Nachweis und/oder zur quantitativen Bestimmung von Nucleinsäuren in einer Probe, umfassend die folgenden Schritte, die in kleinen Volumina zwischen 5 und 50 µl einschließlich vorgenommen werden:
a. Fixieren einer Nucleinsäure durch spezifische Adsorption auf Elektroden, die mit einer Druckfarbe auf Kohlenstoff-Basis siebgedruckt sind,
b. spezifische Hybridisierung einer komplementären Nucleinsäure mit der fixierten Nucleinsäure, wobei die komplementäre Nucleinsäure ein Erkennungsmittel enthält,
c. Zugabe eines zu dem Erkennungsmittel von (b) komplementären Mittels, wobei das komplementäre Mittel an ein Enzym gekoppelt ist,
d. Zugabe eines Substrats des Enzyms auf solche Weise, dass die Einwirkung des Enzyms auf das Substrat zur Bildung einer elektroaktiven Verbindung führt, die durch Messen der Variation des faradischen Stroms nach Anlegen eines Potentials an der Elektrode nachgewiesen werden kann.

2. Verfahren nach Anspruch 1, **dadurch gekennzeichnet, dass** es eine Kaskade von enzymatischen Reaktionen während des Schritts d) vor der Bildung der elektroaktiven Zusammensetzung umfasst.

3. Verfahren nach irgendeinem der Ansprüche 1 oder 2, **dadurch gekennzeichnet, dass** die auf den Elektroden fixierte einzelsträngige Nucleinsäure die Ziel-Nucleinsäure ist.

4. Verfahren nach Anspruch 3, **dadurch gekennzeichnet, dass** es darüber hinaus einen Schritt der Amplifikation vor dem Schritt der Fixierung der Nucleinsäure auf den Elektroden umfasst.

5. Verfahren nach irgendeinem der Ansprüche 1 bis 4, **dadurch gekennzeichnet, dass** der Schritt a) der Fixierung mit einem Fixierungspuffer bewirkt wird, der 1,5 M Ammoniumacetat enthält.

6. Verfahren nach Anspruch 5, **dadurch gekennzeichnet, dass** die Druckfarbe für den Siebdruck eine Mischung von Kohlenstoff und styrolischen Derivaten enthält.

7. Verfahren nach irgendeinem der Ansprüche 1 bis 6, **dadurch gekennzeichnet, dass** das Enzym, das die Bildung der elektroaktiven Verbindung ermöglicht, eine Oxidase ist.

8. Verfahren nach Anspruch 7, **dadurch gekennzeichnet, dass** die Oxidase eine Peroxidase ist.

9. Verfahren nach Anspruch 8, **dadurch gekennzeichnet, dass** die Peroxidase Meerrettichperoxidase ist.

10. Verfahren nach irgendeinem der Ansprüche 8 oder 9, **dadurch gekennzeichnet, dass** das Substrat der Peroxidase ortho-Phenylendiamin OPD ist.

11. Reagenzienkit zur Durchführung eines Verfahrens gemäß irgendeinem der Ansprüche 1 bis 10, **dadurch gekennzeichnet, dass** es enthält:
a. einen 1,5 M Ammoniumacetat enthaltenden Fixierungspuffer, der die spezifische Fixierung der Nucleinsäure auf den Elektroden ermöglicht, die mit einer Druckfarbe auf der Basis von Kohlenstoff siebgedruckt sind,
b. einen Hybridisierungspuffer, der die spezifische Hybridisierung der komplementären Nucleinsäure mit der auf den Elektroden fixierten Nucleinsäure ermöglicht,
c. ein Erkennungsmittel, um die komplementäre Nucleinsäure zu markieren,
d. ein zum Erkennungsmittel (c) komplementäres Mittel, das an ein Enzym gekoppelt ist, und
e. ein Substrat des Enzyms, das zur Bildung einer elektroaktiven Verbindung führt, welche eine Messung der Variation des faradischen Stroms der Lösung ermöglicht.

12. Verfahren zum Nachweis von Viren in einer biologischen Probe, **dadurch gekennzeichnet, dass** es die folgenden Schritte umfasst:
a. eine spezifische Amplifikation der DNA der biologischen Probe, bevorzugt mittels PCR unter Verwendung von spezifischen Primern des gesuchten Virus,
b. die Isolierung und Analyse der durch Amplifikation erhaltenen DNA durch ein Verfahren nach irgendeinem der Ansprüche 1 bis 10 oder unter Verwendung eines Kits nach Anspruch 11.

13. Verfahren zum Nachweis von mehreren verschiedenen Organismen in einer Probe, **dadurch gekennzeichnet, dass** es die folgenden Schritte umfasst, die in kleinen Volumina zwischen 5 und 50 µl einschließlich vorgenommen werden:
a. man bewirkt eine fakultative Amplifikation und die Fixierung der ZielNucleinsäuren der verschiedenen Organismen durch spezifische Adsorption auf der Elektrode, die mit einer Druckfarbe auf Kohlenstoff-Basis siebgedruckt ist,
b. man bewirkt die Hybridisierung mit den zu den Zielen komplementären Nucleinsäuren, wobei jede von ihnen an ein verschiedenes Erkennungsmittel gebunden ist,
c. man gibt die zu den Erkennungsmitteln komplementären Mittel dazu, die an verschiedene enzymatische Marker gekoppelt sind,
d. man gibt die verschiedenen Substrate der Enzyme dazu, um die verschiedenen elektroaktiven Verbindungen zu erzeugen,
e. man misst den faradischen Strom, der den verschiedenen elektroaktiven Verbindungen auf der Oberfläche der Elektrode entspricht, indem man das spezifische Potential jeder Verbindung anlegt.


### LISTAGE DES SÉQUENCES

<110> ARGÈNE SA
<120> KIT ÉLECTROCHIMIQUE DE DÉTECTION D'ACIDES NUCLÉIQUES
<130> D18496
<140> FR 00 02323
   <141> 2000-02-24
<160> 3
<170> PatentIn Ver. 2.2
<210> 1
   <211> 25
   <212> ADN
   <213> HCMV AD169
<400> 1
   ggatccgcat ggcattcacg tatgt 25
<210> 2
   <211> 25
   <212> ADN
   <213> HCMV AD169
<400> 2
   gaattcagtg gataacctgc ggcga 25
<210> 3
   <211> 21
   <212> ADN
   <213> HCMV AD169
<400> 3
   gccaccgcag atagtaagcg c 21